# EUROPÄISCHE PATENTSCHRIFT

(11) **EP 0 621 785 B1**
(45) Veröffentlichungstag und Bekanntmachung des Hinweises auf die Patenterteilung: **04.12.1996**
(21) Anmeldenummer: 92920642.3
(22) Anmeldetag: 29.09.1992
(51) Int. Cl.: A61K 33/40

(54) **VERWENDUNG VON WÄSSRIGEN LÖSUNGEN ENTHALTEND PEROXODISULFATIONEN ZUR BEHANDLUNG MALIGNER ZELLEN**
USE OF AQUEOUS SOLUTIONS CONTAINING PEROXOBISULPHATE IONS FOR THE TREATMENT OF MALIGNANT CELLS
UTILISATION DE SOLUTIONS AQUEUSES CONTENANT DES IONS DE PEROXOBISULFATE POUR LE TRAITEMENT DE CELLULES MALIGNES

(30) Priorität: 24.01.1992 DE 4201858
(43) Veröffentlichungstag der Anmeldung: 02.11.1994
(73) Patentinhaber: RENSCHLER, Aloys, D-50733 Köln (DE)
(72) Erfinder: RENSCHLER, Aloys, D-5000 Köln 60 (DE); SCHUSTER, Dieter, D-42111 Wuppertal (DE)
(74) Vertreter: Jönsson, Hans-Peter, Dr.Dipl.-Chem.
(86) Internationale Anmeldenummer: EP9202244
(87) Internationale Veröffentlichungsnummer: WO9314774

(56) Entgegenhaltungen:
- DE-A- 3 528 379
- FR-A- 2 316 923
- CHEMICAL ABSTRACTS, vol. 96, 1982, Columbus, Ohio, US; abstract no. 173681h; VANNIKOV L.L.: 'ANTIHYPOXIC EFFECT OF PSN', Seite 2, Spalte 1
- CHEMICAL ABSTRACTS, vol. 67, 1967, Columbus, Ohio, US; abstract no. 8451b; A.M. LOKSHINA ET AL.: 'THE RADIOSENSITIZING ACTION OF SODIUM PERSULFATE', Seite 787, Spalte 2
- Derwent Publications Ltd., London, GB; AN 86026462(04)

## Beschreibung

Die Erfindung betrifft die Verwendung von wäßrigen Lösungen enthaltend Peroxodisulfationen in einer Menge von 10⁻⁵ bis 10⁻¹¹ g/l zur Herstellung von Arzneimitteln zur Behandlung maligner Zellen des menschlichen und tierischen Körpers. Beschrieben wird weiterhin ein Verfahren zur Herstellung des Mittels, eine Vorrichtung zur Durchführung des Verfahrens.

Bereits 1959 hat O. Warburg in "Partielle Anaerobiose der Krebszellen und Wirkung der Röntgenstrahlen auf Krebszellen" ("Die Naturwissenschaften", Heft 2, 1959, Seiten 25 ff) festgestellt, daß Wasserstoffperoxid eine spezifische Schädigung von Krebszellen hervorrufen kann. Wasserstoffperoxid wirkt in hohen Konzentrationen als Zellgift. In geringen Konzentrationen, in denen es unter anderem als Zwischenprodukt der Atmungskette der Zellen vorkommt, wird es durch das in gesunden Zellen enthaltene Enzym Katalase abgebaut. Das Enzym Katalase ist jedoch in Krebszellen nur in deutlich verminderter Konzentration vorhanden. Demnach beruht die therapeutische Wirkung von Röntgenstrahlen darauf, daß Wasserstoffperoxid gebildet wird, das in gesunden Zellen durch Katalase abgebaut wird, während es auf Krebszellen die gewünschte schädigende Wirkung hat. Neben dieser Wirkung treten jedoch bei dieser Therapie Nebenwirkungen auf, die auf den bekannten Wechselwirkungsprozessen ionisierender Strahlung mit organischem Gewebe beruhen und den Patienten stark belasten. Die unmittelbare Zufuhr von Wasserstoffperoxid über die Blutbahn zu den zu behandelnden Zellen ist nicht möglich, da die Katalase des Blutserums das Zellgift Wasserstoffperoxid abbaut, bevor es die zu therapierenden Zellen erreicht.

Aus der DE 35 28 379 A1 sind Mittel zur Behandlung wäßriger Systeme sowie zur Regenerierung von Körperzellen, insbesondere Hautzellen, bekannt, bei denen es sich um ein wäßriges Medium mit einem Gehalt an Peroxodisulfationen handelt. Die Mittel werden u.a. zur Regenerierung eingesetzt, d.h. zur Wiederherstellung von Körperzellen, insbesondere Hautzellen. Es wird die topische Anwendung bei Psoriasis und diabetischer Gangrene beschrieben.

Die DE 36 27 759 A1 offenbart pharmazeutische Zusammensetzungen zum Inaktivieren bakterieller Endotoxine, die als Wirkstoff eine nicht toxische, wasserlösliche, pharmazeutisch akzeptable Peroxodiphosphat-Verbindung enthalten.

Aus DERWENT-Abstract 86-02462/04 = PT 80 707 A ist bekannt, daß Derivate der Peroxodiphosphonsäure bei oraler oder systemischer Administration die Entwicklung von Tumorzellen inhibieren. Die Verbindung wird zu einer Salzlösung in einem Phosphatpuffer bei pH 7 bis 7,4 gegeben oder in Form von beschichteten Tabletten, die nicht im Bereich des Magens, sondern durch Einfluß der Intestinalflüssigkeit desintegrieren.

Demgegenüber besteht die Aufgabe der vorliegenden Erfindung in der Bereitstellung und der Verwendung eines spezifisch wirkenden Mittels zur Behandlung maligner Zellen. Die vorgenannten Aufgaben werden mit den Merkmalen der unabhängigen Patentansprüche gelöst.

Eine erste Ausführungsform der Erfindung besteht somit in der Verwendung von wäßrigen Lösungen enthaltend Peroxodisulfationen zur Herstellung von Arzneimitteln zur Behandlung maligner Zellen des menschlichen oder tierischen Körpers.

Eine weitere Ausführungsform der vorliegenden Erfindung besteht somit in der Herstellung der genannten Lösungen, die beispielsweise durch aktinische Bestrahlung von wäßrigen Lösungen, die Anionen von Sauerstoffsäuren des Schwefels, insbesondere Sulfationen und/oder Peroxodisulfationen, enthalten, mit einem pH-Wert von 2 bis 7 erhältlich sind.

Die Konzentration an Peroxodisulfationen in den erfindungsgemäß einzusetzenden wäßrigen Lösungen ist in der Regel äußerst gering, da die Peroxodisulfationen bei hoher Konzentration toxisch wirken. Demgemäß ist es in einer weiteren Ausführungsform der vorliegenden Erfindung bevorzugt, daß die Lösungen 10⁻⁵ bis 10⁻¹¹ g/l, insbesondere 10⁻⁷ bis 10⁻¹¹ g/l Peroxodisulfationen enthalten. Die Konzentration an Peroxodisulfationen sollte 10⁻⁴ g/l nicht überschreiten.

Mit Mitteln dieser Art kann erreicht werden, daß die Zellen des zu behandelnden Zellgewebes über die Blutbahn erreicht werden, bevor sie durch körpereigene Enzyme wie Katalase zersetzt werden. Der gewünschte Abbau der Mittel findet erst nach Anreicherung in den Zellen statt, so daß Wasserstoffperoxid intrazellulär erzeugt wird und dort die gewünschte Wirkung hervorruft. An die Mittel zu stellende Voraussetzungen sind deshalb, daß sie von der Katalase nicht oder nur in geringem Maße angegriffen werden und sich ausreichend langsam in der gewünschten Weise abbauen, so daß die intrazelluläre Entstehung des Wasserstoffperoxids hier sichergestellt ist.

In einer bevorzugten Ausführungsform der vorliegenden Erfindung ist das Mittel erhältlich durch Bestrahlung mit Licht einer Wellenlänge von 190 bis 700 nm, insbesondere UV-Licht. Die Bestrahlungsdauer und -intensität kann beispielsweise eingestellt werden mit Hilfe der erfindungsgemäßen Vorrichtung durch Verdünnung und Bestrahlung.

In einer weiteren bevorzugten Ausführungsform der vorliegenden Erfindung wird davon ausgegangen, daß die verdünnten wäßrigen Lösungen vor der Bestrahlung Anionen von Sauerstoffsäuren des Schwefels in einer Menge von 10⁻² bis 10⁻⁴ g/l enthalten. Da auch nach Abschluß der Bestrahlung von Sulfationen enthaltenden Lösungen noch eine gewisse Menge an Sulfationen erhalten bleibt, ist es bevorzugt, daß das Mittel zur Behandlung maligner Zellen nach der Bestrahlung Sulfationen in einer praktisch nicht nachweisbaren Menge, d.h. höchstens etwa 10⁻⁶ g/l, enthält.

Durch die oben erwähnte aktinische Bestrahlung werden eine Fülle von Reaktionsprodukten aus den Anionen von Sauerstoffsäuren des Schwefels gebildet. Unter anderem konnten Peroxodisulfationen nachgewiesen werden.

Die einzusetzenden Lösungen sind relativ instabil, so daß diese möglichst umgehend dem Patienten verabreicht werden sollten. In einer besonders bevorzugten Ausführungsform der vorliegenden Erfindung ist es jedoch bevorzugt, den Mitteln Stabilisatoren für Peroxidverbindungen zuzusetzen. Besonders bevorzugt im Sinne der vorliegenden Erfindung ist Ethanol als Stabilisator, insbesondere in einer Menge von 15 Gew.-%, bezogen auf die wäßrige Lösung. Darüberhinaus hat sich auch Wasserstoffperoxid als Stabilisator für die einzusetzenden Mittel erwiesen. Bedingt durch die Toxizität ist es jedoch hier erforderlich, geringe Mengen, beispielsweise weniger als 1,5 Gew.%, bezogen auf die wäßrige Lösung einzusetzen.

Zur oralen Applikation ist es besonders bevorzugt, den pH-Wert des erfindungsgemäßen Mittels im Bereich kleiner 7, insbesondere 4 bis 5, einzustellen. Daneben ist aber in gleicher Weise auch die sublinguale oder parenterale, nämlich intratumorale, intramuskuläre oder intravenöse Applikation möglich.

In die Blutbahn eingebrachten wäßrigen Lösungen dieser Mittel in geeigneter Konzentration können zunächst vom Blut ohne nennenswerten Abbau transportiert werden. Sie treten nach und nach in das Innere sowohl gesunder als auch kranker Zellen ein. In gesunden Zellen werden durch hydrolytische Zersetzung entstehende Wasserstoffperoxid-Moleküle durch das in ausreichender Menge vorhandene Enzym Katalase sofort ohne Schädigung der Zellen abgebaut. In den malignen Zellen mit vermindertem Gehalt dieses Enzyms kann eine Anreicherung der Lösung stattfinden, die schließlich zur Vernichtung der malignen Zellen führt oder wenigstens deren Teilung verzögert oder vollständig verhindert.

Die Herstellung medizinisch verwendbarer Lösungen erfolgt vorzugsweise mit einer nachfolgend beschriebenen Vorrichtung, wobei verdünnte Lösungen, die Anionen von Sauerstoffsäuren des Schwefels, insbesondere Schwefelsäurelösung, in destilliertem Wasser ein System von Röhren durchströmen und dabei kontinuierlich verdünnt und aktinisch, beispielsweise mit UV-Licht, bestrahlt werden.

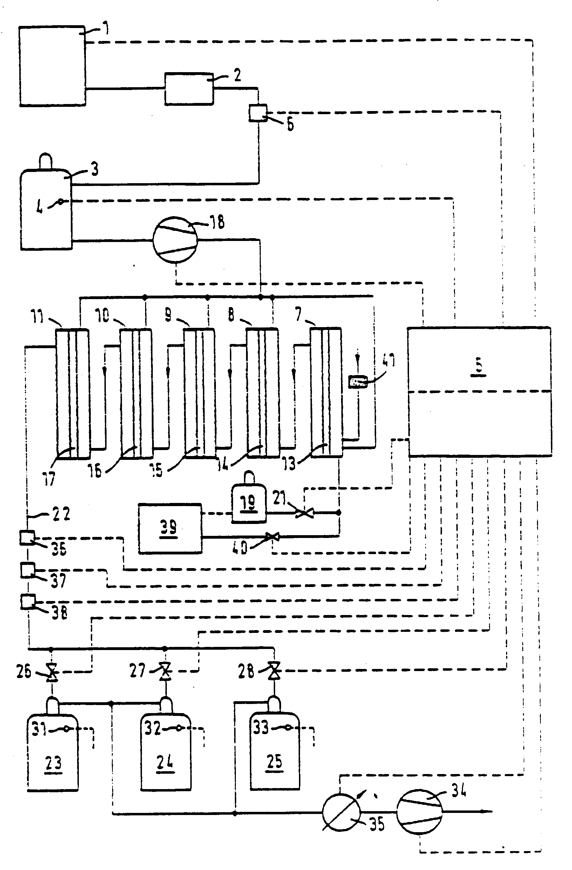

Einzelheiten diese Art der Herstellung der Mittel sollen anhand der Figur erläutert werden.

Mit 1 ist ein Block dargestellt, in welchem die Herstellung des hochreinen Wassers stattfindet, von dem die Erfindung ausgeht. Zweckmäßig erfolgt die Herstellung des hochreinen Wassers, indem die folgenden Verfahrensschritte nacheinander ausgeführt werden: Demineralisierung, Destillation, UV-Bestrahlung des Dampfes während der Destillation. Diese Bestrahlung hat den Zweck, das Wasser völlig keimfrei zu machen.

Das hochreine Wasser wird über einen Filter 2 einem Vorratsgefäß 3 zugeführt. Dieses Vorratsgefäß 3 ist mit einem Füllstandssensor 4 ausgerüstet, diessen Signale einem Kontroll- und Steuergerät 5 zugeführt werden. In Abhängigkeit vom Füllstand erfolgt die Herstellung des hochreinen Wassers. Dazu ist der Block 1 über eine Steuerleitung mit der Kontroll- und Steuereinrichtung 5 verbunden.

Der Leitwert des hochreinen Wassers wird durch Messungen kontrolliert. Dazu ist in die vom Block 1 zum Vorratsgefäß 3 führende Leitung ein Leitwertmeßgerät 6 eingeschaltet. Auch dieses liefert seine Signale an die zentrale Kontroll- und Steuereinheit 5. Das hochreine Wasser soll einen extrem kleinen Leitwert (gemessen in Siemens, S) haben, und zwar im µS-Bereich.

Die eigentliche Herstellung des Mittels findet in den Behältern 7 bis 11 statt. In diesen Behältern befindet sich jeweils eine Bestrahlungsröhre 13 bis 17, von denen Licht im IR- bis UV-Bereich abgestrahlt wird, beispielsweise einen UV-Wasserklärer (UV-Sterilizer) der Fa. Wiegandt (Krefeld) 30 W, 220/230 V, etwa 50 Hz, Länge ca. 1 m.

Der von den Behältern 7 bis 11 und den darin axial angeordneten Bestrahlungsröhren 13 bis 17 gebildete Raum mit kreisringförmigem Querschnitt wird von der verdünnten wäßrigen Löung durchströmt. Die Dimensionen sind etwa so gewählt, daß sich etwa 6 ml Wasser pro 1 cm Länge in den Behältern 7 bis 11 befinden.

Die Art und Weise der Spannungsversorgung der Röhren 13 bis 17 ist im einzelnen nicht dargestellt. Zweckmäßig wird sie ebenfalls von der Kontroll- und Steuereinheit geschaltet.

Um eine Strömung und Verdünnung zu erzielen, wird jedem der Behälter 7 bis 11 hochreines Wasser aus dem Vorratsbehälter 3 über die Pumpe 18 - vorzugsweise einer Schlauchpumpe - zugeführt. Die Strömungsgeschwindigkeit der zu bestrahlenden Flüssigkeit in den Behältern 7 bis 11 soll relativ klein sein. Das wird dadurch erreicht, daß in den einzelnen Zuleitungen zu den Behältern 7 bis 11 eine Strömung eingestellt wird, die bei 100 ml/h liegt. Die Geschwindigkeit hängt allgemein von der Art, der Zahl und der Größe der Röhren ab. Wesentlich ist, daß die Lösung ihre oxidierende Eigenschaft erhält.

Dem in Strömungsrichtung ersten Behälter 7 wird zusätzlich eine bestimmte Menge beispielsweise an Sulfationen in wäßriger Lösung aus dem Vorratsbehälter 19 zugeführt. Die Zufuhr erfolgt diskontinuierlich und wird vom Ventil 21 gesteuert. Die Betätigung dieses Ventils erfolgt wieder mit Hilfe der Kontroll- und Steuereinheit 5.

Nach der Zugabe der bestimmten Menge der Sulfationen beginnt der der Herstellung des Mittels dienende Bestrahlungs- und Verdünnungsprozeß.

Die in den Behälter 7 gelangte Lösung wird mit dem darin einströmenden hochreinen Wasser verdünnt. Nach dem Durchströmen des ersten Behälters 7 tritt die Lösung in den zweiten Behälter 8 ein. Dort werden Bestrahlung und Verdünnung fortgesetzt, indem weiteres hochreines Wasser zugeführt wird. Dieser Vorgang setzt sich in den weiteren Behältern 9 bis 11 fort. Aus dem letzten Behälter 11 tritt die Lösung aus und wird über die Leitung 22 den Vorratsbehältern 23, 24, 25 in der noch zu beschreibenden Weise zugeführt.

Die Vorratsbehälter 23, 24, 25 sind über die Ventile 26, 27, 28 an die Leitung 22 angeschlossen. Diese Ventile sind zweckmäßig als Elektromagnetventile ausgebildet, so daß sie mit Hilfe der Kontroll- und Steuereinheit 5 betätigbar sind.

Weiterhin sind die Vorratsbehälter 23, 24, 25 mit Füllstandssensoren 31, 32, 33 ausgerüstet, welche in im einzelnen nicht dargestellter Weise ebenfalls mit der Kontroll- und Steuereinheit 5 verbunden sind. Schließlich ist an die Behälter 23, 24, 25 die Vakuumpumpe 34 angeschlossen, mit der ein Unterdruck von etwa 200 bis 300 mbar erzeugt wird. Zur Überwachung dieses Druckes ist das Meßgerät 35 vorgesehen, dessen Signale ebenfalls der Kontroll- und Steuereinheit 5 zugeführt werden.

In der Leitung 22 befinden sich Sensoren bzw. Meßgeräte, mit denen die Temperatur (36) der pH-Wert (37) und der Leitwert (38) überwacht werden können. Die von den Sensoren 36, 37, 38 gelieferten Signale werden wieder der Kontroll- und Steuereinheit 5 zugeführt.

Nach der Zufuhr der bestimmten Menge an Sulfationen aus dem Vorratsbehälter 19 in den ersten Bestrahlungsbehälter 7 beginnt der Bestrahlungs- und Verdünnungsprozeß. Wird eine H₂SO₄-Lösung verwendet, dann ist es zweckmäßig, wenn diese mit einer Konzentration von 10⁻² g/l vorliegt.

In der unmittelbar nach dem Beginn des Herstellungsprozesses aus dem Behälter 11 austretenden Lösung ist die dem Behälter 7 zugegebene Substanz (H₂SO₄) noch nicht vorhanden. Es wird deshalb solange gewartet, bis anhand der Meßgeräte 36 bis 38 erkennbar ist, daß die aus dem Behälter 11 austretende Flüssigkeit Sulfationen enthält. Bis zu diesem Zeitpunkt sind die Ventile 27 und 28 geschlossen und das Ventil 26 geöffnet. Die durch dieses Ventil 26 austretende Flüssigkeit wird verworfen.

Das Ventil 26 wird erst geschlossen wen die Sulfationenkonzentration in der aus dem letzten Bestrahlungsgefäß 11 austretenden Flüssigkeit etwa 10⁻⁵ bis 10⁻⁶ g/l beträgt. Durch Öffnen des Ventils 27 wird erreicht, daß diese Lösung in den Vorratsbehälter 24 strömt. Die Konzentration an Sulfationen wird danach ständig abnehmen. Zweckmäßig ist es, die Ventile 26, 27, 28 so zu steuern, daß in die Vorratsbehälter 23, 24, 25 Lösungen mit unterschiedlichen Konzentrationen gelangen, so daß z.B. im Behälter 23 eine Sulfationenkonzentration von etwa 10⁻⁵ g/l, im Vorratsbehälter 24 eine Konzentration von etwa 10⁻⁶ g/l und im Vorratsbehälter 25 eine Konzentration von 10⁻⁷ g/l vorliegt, während die Konzentration an Peroxodisulfationen in gleicher Weise zunimmt. Die im Vorratsbehälter 24 eingeströmte Lösung kann ebenfalls als Ausgangslösung verwendet werden, indem sie dem ersten Behälter 7 (anstelle der im Vorratsbehälter 19 befindlichen Flüssigkeit) zugeführt wird. Der notwendige Verdünnungsgrad ist dann schneller erreichbar. Im Vorratsbehälter 25 wird das eigentliche Mittel gesammelt. Die behandelte Lösung strömt darin ein, solange sie eine Konzentration an Peroxodisulfationen von etwa 10⁻⁵ bis 10⁻¹¹ g/l hat.

Der eigentliche Durchlaufprozeß dauert ca. 15 Stunden. Ca. 4,2 l des erfindungsgemäßen Mittels liegen danach vor.

Die beschriebene Vorrichtung erlaubt eine diskontinuierliche Betriebsweise. Um eine kontinuierliche Herstellung des erfindungsgemäßen Mittels zu ermöglichen, ist es zweckmäßig, eine weitere Vorrichtung der beschriebenen Art einzusetzen und diese an den ersten Bestrahlungsbehälter 7 anzuschließen. In der Figur ist eine derartige weitere Vorrichtung lediglich als Block 39 dargestellt, die über das Ventil 40 an den Vorratsbehälter 7 anschließbar ist. Hat die Ausgangssubstanz der ersten Anlage 39 eine geeignete Konzentration (z.B. 10⁻⁶ g/l), dann erfolgt über das Ventil 40 die Verbindung mit der Zulieferanlage 39. Über nicht dargestellte Leitungen wird die Ausgangssubstanz der Zulieferanlage 39, solange die Konzentration von 10⁻⁶ g/l noch nicht erreicht ist, im Behälter 23 gesammelt. Bei einer Konzentration der aus dem Behälter 11 ausströmenden Lösung von etwa 10⁻⁹ g/l muß der erste Bestrahlungsbehälter der Zulieferanlage 39 mit Ausgangslösung aus dem Behälter 19 versorgt werden. Die beiden Anlagen werden dann so lange voneinander getrennt bis die die Zulieferanlage verlassende Lösung die für die Zuschaltung erforderliche Konzentration hat. Der Behälter 24 kann bei dieser Betriebsweise entfallen. In den Behälter 25 strömt wieder das erfindungsgemäße Mittel.

Ein weiterer Vorteil des Einsatzes einer Zulieferanlage 39 besteht darin, daß der Bestrahlungs- und Verdünnungsprozeß stets von einer neuen (nicht bereits behandelten) Substanz ausgeht.

Für die Herstellung der erfindungsgemäßen Mittel sind die physikalischen Bedingungen, unter denen der Verdünnungs- und Bestrahlungsprozeß abläuft, von besonderer Bedeutung. Die Temperatur soll möglichst niedrig, beispielsweise beim Raumtemperatur, oder in der Größenordnung von 40 °C liegen. Diese Temperatur wird mit Hilfe des Meßgerätes 36 überwacht. Steigt die Temperatur wegen der von den Bestrahlungsröhren 13 bis 17 erzeugten Wärme, müssen nicht dargestellte Kühleinrichtungen, z.B. ein Ventilator, eingeschaltet werden.

Von der Vakuumpumpe 34 wird ein Unterdruck erzeugt, der einige hundert Millibar beträgt. Dieser Unterdruck bewirkt zum einen die erwünschte Förderung der Lösung entlang der Vorrichtung. Weiterhin gibt die Lösung gelöste Gase (CO₂, O₂) oder dergleichen ab. Schließlich kann aufgrund der Wirkung der Vakuumpumpe 34 dem ersten Bestrahlungsbehälter 7 Gas zugeführt werden, welches in sämtlichen Behältern eine Turbulenz erzeugt. Zweckmäßig wird reine Luft zugeführt. Der Reinigung der zugeführten Luft dient der Filter 41.

Die pH-Werte der Mittel liegen bei Verwendung von verdünnter Schwefelsäure als Ausgangssubstanz je nach Konzentration an Sulfationen bei etwa 4,5 bis 6,5. Der Leitwert liegt zwischen 100 und 400 µS.

Mit Hilfe der Kontroll- und Steuereinheit 5 ist es möglich, den Ablauf des Herstellungsprozesses zu automatisieren. Die Meßgeräte 6, 35, 36, 37, 38 sind über Signalleitungen mit der Einheit 5 verbunden. Die gelieferten Signale werden aufbereitet und dienen der Steuerung der dem Ablauf des Herstellungsprozesses dienenden Geräte und Bauteile (Mittel 1 bis 4 zur Herstellung des Ausgangsproduktes, Förderpumpe 18, Ventile 21, 26, 27, 28, 39, Vakuumpumpe 34), welche ihrerseits über Steuerleitungen mit der Einheit 5 verbunden sind. Ist der dargestellten Anlage eine Zulieferanlage 39 vorgelagert, dann muß auch die Konzentration des Ausgangsproduktes dieser Zulieferanlage überwacht und das Ventil 40 von der Einheit 5 gesteuert werden.

### Beispiele

### Beispiel 1

Bei dem Patienten A, der seit 1988 wegen eines rezidivierenden malignen Blasentumors behandelt wurde, kam es trotz wiederholter Elektroresektionen immer wieder zu einem Tumor-Rezidiv Der behandelnde Urologe erwog wegen Perforationsgefahr und/oder der Gefahr der Tumor-Metastasierung eine totale Blasenresektion. Mitte Mai 1988 erfolgte erstmalig die Behandlung mit einer verdünnten wäßrigen Lösung, die entsprechend der vorliegenden Erfindung hergestellt wurde und 10⁻⁶ g/l Peroxodisulfationen enthielt. Der Patient nahm täglich 3x1 Teelöffel ein. Bei einer 8 Wochen später durchgeführten Cystoskopie-Kontrolle stellte sich die Blasenschleimhaut völlig unauffällig dar. Seit dieser Zeit wurden regelmäßig alle 3-4 Monate Spiegelkontrollen durchgeführt. Ein Tumor-Rezidiv konnte nicht mehr nachgewiesen werden. Es handelte sich um ein rezidivierendes papilläres uroepitheliäres Carcinom.

### Beispiel 2

Bei einer Patientin B ergab eine Untersuchung den hochgradigen Verdacht auf ein ductales Carcinom in der linken Brust. Eine Stanzpunktion des verdächtigen Knotens bestätigte die Diagnose. Die empfohlene Mammaamputation wurde von der Patientin abgelehnt. Die regelmäßige Einnahme von täglich 3x1 Teelöffel einer verdünnten wäßrigen Lösung, die entsprechend der vorliegenden Erfindung hergestellt wurde und 10⁻⁶ g/l Peroxodisulfationen enthielt, führte zu einer langsamen Rückbildung des Knotens. Halbjährlich durchgeführte mammographische Kontrollen bestätigten den palpatorischen Befund. Die letzte radiologische Kontrolle nach zweijähriger Behandlungsdauer ergab folgende Beurteilung: Histologisch gesichertes intraductales Carcinom der linken mamma unter alternativer Therapie. Im Vergleich zur Voruntersuchung kein Hinweis auf malignom-typische Strukturen der linken mamma.

### Beispiel 3

Bei dem Patienten C wurde ein hepatozelluläres Carcinom mit ausgedehnter Lungenmetastasierung festegestellt. Nach Durchführung der Therapie analog den Beispielen 1 und 2 ergab eine durchgeführte Thorax-Übersichtsaufnahme eine deutliche Rückbildung der Lungenrundherde, die allenfalls noch angedeutet zur Darstellung kamen. Beurteilung: Remission bei bekannter Lungenmetastasierung.

### Beispiel 4

Bei der Patientin D wurde im Januar 1989 ein Mammacarcinom im äußeren oberen Quadranten der linken mamma festgestellt. Eine nach einem Monat durchgeführte Kontrollaufnahme ergab eine Größenzunahme des Tumors. Hierauf erfolgt eine Therapie mit täglich 3x1 Teelöffel einer verdünnten wäßrigen Lösung, die entsprechend der vorliegenden Erfindung hergestellt wurde und 10⁻⁸ g/l Peroxodisulfationen enthielt. Regelmäßig durchgeführte Kontrollmammographien in 4-5-monatigen Abständen ergaben eine langsame Rückbildung des Tumors. Nach 9 Monaten war der Herdschatten eindeutig auf nahezu die Hälfte zurückgegangen. Die Kontroll-Mammographie vom Oktober 1991 zeigte nur noch eine geringgradige Gewebsverdichtung, die nur als Narbengewebe imponiert.

## Patentansprüche

1. Verwendung von wäßrigen Lösungen enthaltend Peroxodisulfationen in einer Menge von 10⁻⁵ bis 10⁻¹¹ g/l zur Herstellung von Arzneimitteln zur Behandlung maligner Zellen des menschlichen oder tierischen Körpers.

2. Verwendung nach Anspruch 1, dadurch gekennzeichnet daß die Lösungen 10⁻⁷ bis 10-¹¹g/l Peroxodisulfationen enthalten.

3. Verwendung nach Anspruch 1 oder 2, dadurch gekennzeichnet, daß die Lösungen durch aktinische Bestrahlung von wäßrigen Lösungen mit einem pH-Wert von 2 bis 7, die Anionen von Sauerstoffsäuren des Schwefels enthalten, erhalten werden.

4. Verwendung nach einem der Ansprüche 1 bis 3, dadurch gekennzeichnet, daß die wäßrigen Lösungen vor der Bestrahlung 10⁻² bis 10⁻⁴ g/l Sulfationen enthalten.

5. Verwendung nach einem der Ansprüche 1 bis 3, dadurch gekennzeichnet, daß die Lösungen Stabilisatoren, insbesondere Ethanol und/oder Wasserstoffperoxid in einer Menge von bis zu 15 Gew.-% Ethanol und bis zu 1,5 Gew.% Ethanol und 1,5 Gew.% Wasserstoffperoxid, bezogen auf die Lösung, enthalten.

6. Verwendung nach einem der Ansprüche 1 bis 5, dadurch gekennzeichnet, daß der pH-Wert auf einen Bereich von 4 bis 5 eingestellt ist.

7. Verwendung nach einem der Ansprüche 3 bis 6, dadurch gekennzeichnet, daß mehrere, insbesondere wenigstens 5 Bestrahlungsbehälter (7, 8, 9, 10 und 11) vorgesehen sind, wobei dem ersten Bestrahlungsbehälter (7) eine bestimmte Menge an Anionen von Sauerstoffsäuren des Schwefels zugegeben wird und zur Durchführung des Verdünnungsprozesses jedem der Bestrahlungsbehälter (7, 8, 9, 10 und 11) hochreines Wasser zugeführt wird.

8. Verwendung nach einem der Ansprüche 3 bis 7, dadurch gekennzeichnet, daß die Bestrahlung und Verdünnung bei Unterdruck durchgeführt wird und/oder dem ersten Bestrahlungsbehälter (7) zusätzlich ein Inertgas zur Erzeugung von Turbulenzen zugeführt wird und/oder daß die die Behälter (7, 8, 9, 10 und 11) durchströmende Flüssigkeit auf einer Temperatur im Bereich der Zimmertemperatur oder 30 bis 50 °C, insbesondere 40 °C, gehalten wird.

9. Verwendung nach einem der Ansprüche 3 bis 8, dadurch gekennzeichnet, daß mehrere, insbesondere wenigstens fünf, zylindrische Behälter (7, 8, 9, 10 und 11) mit axial darin angeordneten Bestrahlungsröhren (13, 14, 15, 16 und 17) vorgesehen sind.

10. Verwendung nach einem der Ansprüche 3 bis 9, gekennzeichnet durch Einrichtungen (1, 2, 3 und 4) zur Herstellung und Bereithaltung von hochreinem Wasser.

11. Verwendung nach einem der Ansprüche 3 bis 10, dadurch gekennzeichnet, daß an dem in Strömungsrichtung ersten Behälter (7) über ein Ventil (21) ein Vorratsbehälter (19) mit einer Lösung, enthaltend Anionen von Sauerstoffsäuren des Schwefels, angeschlossen ist.

12. Verwendung nach einem der Ansprüche 3 bis 11, dadurch gekennzeichnet, daß an den ersten Behälter (7) eine der Zuführung von Luft dienende Leitung mit einem Filter (41) angeschlossen ist.

13. Verwendung nach einem der Ansprüche 3 bis 12, dadurch gekennzeichnet, daß an dem in Strömungsrichtung letzten Behälter (11) eine Leitung (22) angeschlossen ist, die den letzten Behälter (11) mit mehreren, insbesondere drei Vorratsbehältern (23, 24, 25) verbindet.

14. Verwendung nach einem der Ansprüche 3 bis 13, dadurch gekennzeichnet, daß in der Leitung (22) Sensoren und/oder Meßgeräte (36, 37, 38) zur Überwachung der Eigenschaften der den Behälter (11) verlassenden Lösung angeordnet sind.

15. Verwendung nach einem der Ansprüche 3 bis 14, dadurch gekennzeichnet, daß die Vorratsbehälter (23, 24, 25) über Magnetventile (26, 27, 28) an die Leitung (22) angeschlossen sind.

16. Verwendung nach einem der Ansprüche 3 bis 15, dadurch gekennzeichnet, daß an die Vorratsbehälter (23, 24, 25) eine Vakuumpumpe (34) angeschlossen ist.

17. Verwendung nach einem der Ansprüche 3 bis 16, dadurch gekennzeichnet, daß eine zentrale Kontroll- und Steuereinheit (5) vorgesehen ist, die über Signalleitungen mit Meßgeräten (6, 35, 36, 37, 38) und Füllstandssensoren (4, 31, 32, 33) verbunden ist.

18. Verwendung nach einem der Ansprüche 3 bis 17, dadurch gekennzeichnet, daß die dem Ablauf des Bestrahlungs- und Verdünnungsprozesses dienenden Geräte (Mittel 1 bis 4 zur Herstellung des Ausgangsproduktes, Förderpumpe 18, Ventile 21, 26, 27, 28, 39, Vakuumpumpe 34) über Steuerleitungen mit der zentralen Kontroll- und Steuereinheit (5) verbunden sind.

19. Verwendung nach einem der Ansprüche 3 bis 18, dadurch gekennzeichnet, daß zwei Bestrahlungs- und Verdünnungsanlagen hintereinandergeschaltet sind.

20. Verwendung von wäßrigen Lösungen enthaltend Sulfationen in einer Menge von höchstens 10⁻⁶g/l zur Herstellung von Arzneimitteln zur Behandlung maligner Zellen des menschlichen oder tierischen Körpers.

## Claims

1. Use of aqueous solutions containing peroxodisulfate ions in an amount of from 10⁻⁵ to 10⁻¹¹ g/l for the preparation of medicaments for treating malignant cells of the human or animal body.

2. The use according to claim 1, characterized in that said solutions contain from 10⁻⁷ to 10⁻¹¹ g/l of peroxodisulfate ions.

3. The use according to claim 1 or 2, characterized in that said solutions are obtained by subjecting aqueous solutions having a pH value of from 2 to 7 and containing anions of oxo acids of sulfur to actinic radiation.

4. The use according to any of claims 1 to 3, characterized in that said aqueous solutions contain from 10⁻² to 10⁻⁴ g/l of sulfate ions prior to being subjected to said radiation.

5. The use according to any of claims 1 to 3, characterized in that said solutions contain stabilizers, in particular ethanol and/or hydrogen peroxide, in amounts of up to 15% by weight of ethanol and up to 1.5% by weight of hydrogen peroxide, based on the solution.

6. The use according to any of claims 1 to 5, characterized in that the pH value is adjusted to a range of from 4 to 5.

7. The use according to any of claims 3 to 6, characterized in that a plurality of, in particular at least five, irradiation vessels (7, 8, 9, 10 and 11) are provided wherein a certain amount of anions of oxo acids of sulfur is added to the first irradiation vessel (7) and high-purity water is supplied to each of the irradiation vessels (7, 8, 9, 10 and 11) for performing the dilution process.

8. The use according to any of claims 3 to 7, characterized in that said irradiation and dilution are performed under reduced pressure and/or an inert gas is additionally supplied to the first irradiation vessel (7) to generate turbulences and/or the liquid flowing through the vessels (7, 8, 9, 10 and 11) is kept at a temperature in the range of room temperature of 30 to 50°C, in particular 40°C.

9. The use according to any of claims 3 to 8, characterized in that a plurality of, in particular at least five, cylindrical vessels (7, 8, 9, 10 and 11) having irradiation tubes (13, 14, 15, 16 and 17) axially arranged therein are provided.

10. The use according to any of claims 3 to 9, characterized by means (1, 2, 3 and 4) for the preparation and storage of high-purity water.

11. The use according to any of claims 3 to 10, characterized in that a supply vessel (19) with a solution containing anions of oxo acids of sulfur is connected via a valve (21) with the first vessel as seen in the flow direction (7).

12. The use according to any of claims 3 to 11, characterized in that a line having a filter (41) and serving for air supply is connected with the first vessel (7).

13. The use according to any of claims 3 to 12, characterized in that a line (22) is connected with the last vessel as seen in the flow direction (11), said line connecting the last vessel (11) with a plurality of, in particular three, supply vessels (23, 24, 25).

14. The use according to any of claims 3 to 13, characterized in that sensors and/or measuring devices (36, 37, 38) are arranged in line (22) for monitoring the properties of the solution leaving the vessel (11).

15. The use according to any of claims 3 to 14, characterized in that the supply vessels (23, 24, 25) are connected with line (22) via magnet valves (26, 27, 28).

16. The use according to any of claims 3 to 15, characterized in that a vacuum pump (34) is connected with the supply vessels (23, 24, 25).

17. The use according to any of claims 3 to 16, characterized in that a central control unit (5) is provided which is connected to measuring devices (6, 35, 36, 37, 38) and filling level sensors (4, 31, 32, 33) via signal lines.

18. The use according to any of claims 3 to 17, characterized in that the devices serving for the performance of the irradiation and dilution process (means 1 to 4 for the preparation of the starting material, feed pump 18, valves 21, 26, 27, 28, 39, vacuum pump 34) are connected with the central control unit (5) via controlling lines.

19. The use according to any of claims 3 to 18, characterized in that two irradiation and dilution devices are used in series.

20. Use of aqueous solutions containing sulfate ions in an amount of not greater than 10⁻⁶ g/l for the preparation of medicaments for treating malignant cells of the human or animal body.

## Revendications

1. Utilisation de solutions aqueuses contenant des ions peroxobisulfate à raison de 10⁻⁵ g / l jusqu'à 10⁻¹¹ g / l pour la préparation de médicaments destinés au traitement de cellules malignes du corps humain ou animal.

2. Utilisation selon la revendication 1, caractérisée en ce que les solutions contiennent de 10⁻⁷ g / l jusqu'à 10⁻¹¹ g / l d'ions peroxobisulfate.

3. Utilisation selon la revendication 1 ou 2, caractérisée en ce que les solutions sont obtenues par irradiation actinique de solutions aqueuses présentant une valeur de pH de 2 jusqu'à 7 et qui contiennent des anions d'acides oxygénés du soufre.

4. Utilisation selon l'une quelconque des revendications 1 à 3, caractérisée en ce que les solutions aqueuses contiennent de 10⁻² g / l jusqu'à 10⁻⁴ g / l d'ions sulfate avant l'irradiation.

5. Utilisation selon l'une quelconque des revendications 1 à 3, caractérisée en ce que les solutions, contiennent des stabilisateurs, en particulier l'éthanol et / ou le peroxyde d'hydrogène jusqu'à 15 % en poids d'éthanol etjusqu'à 1,5 % en poids d'éthanol et 1,5 % en poids de peroxyde d'hydrogène, par rapport à la solution.

6. Utilisation selon l'une quelconque des revendications 1 à 5, caractérisée en ce que le pH présente une valeur ajustée de 4 à 5.

7. Utilisation selon l'une quelconque des revendications 3 à 6, caractérisée en ce que plusieurs, en particulier au moins 5, récipients d'irradiation (7, 8, 9, 10 et 11) sont prévus, au cours de laquelle on ajoute au premier récipient d'irradiation (7) une quantité déterminée d'anions d'acides oxygénés du soufre et on ajoute de l'eau ultrapure à chacun des récipients d'irradiation (7, 8, 9, 10 et 11) pour effectuer la dilution.

8. Utilisation selon l'une quelconque des revendications 3 à 7, caractérisée en ce qu'on réalise l'irradiation et la dilution sous pression réduite et / ou en ce que l'on fournit en outre au premier récipient d'irradiation (7) un gaz inerte pour la production de turbulences et / ou en ce que le liquide traversant les récipients (7, 8, 9, 10 et 11) est maintenu à une température aux environs de la température ambiante ou de 30 °C jusqu'à 50 °C, en particulier de 40 °C.

9. Utilisation selon l'une quelconque des revendications 3 à 8, caractérisée en ce qu'on met en oeuvre plusieurs, en particulier au moins cinq, récipients cylindriques (7, 8, 9, 10 et 11) équipés de tubes d'irradiation qui y sont disposés dedans de manière axiale (13,14, 15, 16 et 17).

10. Utilisation selon l'une quelconque des revendications 3 à 9, caractérisée par les dispositifs (1, 2, 3, 4) pour la production et la mise à disposition d'eau ultrapure.

11. Utilisation selon l'une quelconque des revendications 3 à 10, caractérisée en ce qu'un récipient de stockage (19) rempli d'une solution contenant des anions d'acides oxygénés du soufre est connecté par l'intermédiaire d'une soupape (21) au premier récipient (7) dans la direction de l'écoulement.

12. Utilisation selon l'une quelconque des revendications 3 à 11, caractérisée en ce qu'une canalisation de service pour l'alimentation d'air équipée d'un filtre (41) est connectée au premier récipient (7).

13. Utilisation selon l'une quelconque des revendications 3 à 12, caractérisée en ce qu'une canalisation (22) est connectée au dernier récipient (11) dans la direction de l'écoulement, raccordant le dernier récipient (11) à plusieurs, en particulier à trois, récipients de stockage (23, 24, 25).

14. Utilisation selon l'une quelconque des revendications 3 à 13, caractérisée en ce qu'on dispose des capteurs et / ou des appareils de mesure (36, 37, 38) dans la canalisation (22) afin de contrôler les propriétés de la solution à la sortie du récipient (11).

15. Utilisation selon l'une quelconque des revendications 3 à 14, caractérisée en ce que les recipients de stockage (23, 24, 25) sont connectés à la canalisation (22) par l'intermédiaire de soupapes magnétiques (26, 27, 28).

16. Utilisation selon l'une quelconque des revendications 3 à 15, caractérisée en ce qu'on connecte une pompe à vide (34) aux récipients de stockage (23, 24, 25).

17. Utilisation selon l'une quelconque des revendications 3 à 16, caractérisée en ce qu'on prévoit une unité centrale de contrôle et de réglage (5) qui est connectée, par l'intermédiaire de canalisations de signalisation, aux appareils de mesure (6, 35, 36, 37, 38) et aux capteurs de niveaux (4, 31, 32, 33).

18. Utilisation selon l'une quelconque des revendications 3 à 17, caractérisée en ce que les dispositifs servant au déroulement du procédé d'irradiation et de dilution (moyens 1 à 4 pour la préparation du produit de départ, pompe de transfert 18, soupapes 21, 26, 27, 28, 39, pompe à vide 34) sont connectés par l'intermédiaire de canalisations de réglage, à l'unité centrale de contrôle et de réglage (5).

19. Utilisation selon l'une quelconque des revendications 3 à 18, caractérisée en ce que deux systèmes d'irradiation et de dilution sont disposés l'un après l'autre.

20. Utilisation de solutions aqueuses contenant des ions sulfate à raison de 10⁻⁶ g / l au maximum pour la préparation de médicaments destinés au traitement de cellules malignes du corps humain ou animal.
